# EUROPEAN PATENT SPECIFICATION

(11) **EP 4 021 563 B1**
(45) Date of publication and mention of the grant of the patent: **04.12.2024**
(21) Application number: 20859157.8
(22) Date of filing: 20.08.2020
(51) Int. Cl.: A61N 1/05, A61N 1/36, A61N 1/375

(54) **IMPLANTABLE CARRIER WITH EMBEDDED STABILIZER**
IMPLANTIERBARER TRÄGER MIT EINGEBETTETEM STABILISATOR
SUPPORT IMPLANTABLE AVEC STABILISATEUR INTÉGRÉ

(30) Priority: 29.08.2019 US 201962893330 P
(43) Date of publication of application: 06.07.2022
(73) Proprietor: Cochlear Limited, Macquarie University, NSW 2109 (AU)
(72) Inventor: MALOUF, Christopher, Macquarie University, NSW 2109 (AU); PAWSEY, Nicholas Charles, Macquarie University, NSW 2109 (AU)
(74) Representative: Grünecker Patent- und Rechtsanwälte PartG mbB
(86) International application number: PCT/IB2020/000681
(87) International publication number: WO 2021/038290

(56) References cited:
- WO-A2-2014/127119
- WO-A2-2014/127119
- US-A- 5 545 219
- US-A- 6 074 422
- US-A1- 2007 135 885
- US-A1- 2009 030 483
- US-A1- 2009 030 483
- US-A1- 2017 326 366

## Description

### Background

Medical devices having one or more implantable components, generally referred to herein as implantable medical devices, have provided a wide range of therapeutic benefits to recipients over recent decades. In particular, partially or fully- implantable medical devices such as hearing prostheses (e.g., bone conduction devices, mechanical stimulators, cochlear implants, etc.), implantable pacemakers, defibrillators, functional electrical stimulation devices, and other implantable medical devices, have been successful in performing lifesaving and/or lifestyle enhancement functions and/or recipient monitoring for a number of years.

The types of implantable medical devices and the ranges of functions performed thereby have increased over the years. For example, many implantable medical devices now often include one or more instruments, apparatus, sensors, processors, controllers or other functional mechanical or electrical components that are permanently or temporarily implanted in a recipient. These functional devices are typically used to diagnose, prevent, monitor, treat, or manage a disease/injury or symptom thereof, or to investigate, replace or modify the anatomy or a physiological process. Many of these functional devices utilize power and/or data received from external devices that are part of, or operate in conjunction with, the implantable medical device.
US-A1-2009/030483 discloses an elongate implantable carrier member having an embedded stiffener.
US-A1-2007/135885 discloses a flexible implantable electrode assembly for a stimulating medical device.

### Summary

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes.

In an example, there is an apparatus having: a flexible elongate carrier having a proximal region and being configured to introduce a therapeutic element into a recipient; and a stabilizer permanently embedded in and longitudinally extending through at least the proximal region. The stabilizer is configured to decrease flexibility of the proximal region so as to resist deformation of the proximal region during introduction of the flexible elongate carrier into the recipient. The stabilizer comprises an elastomeric material.

In another example, there is a flexible elongate carrier for introducing a therapeutic element into a recipient. The flexible elongate carrier includes a first elastomeric body material having a first hardness and a stabilizer extending through at least a portion of the first elastomeric body material. The stabilizer includes a second elastomeric body material having a second hardness greater than the first hardness.

In yet another example, there is a method comprising: forming a carrier at least partially from a first elastomeric body material having a first hardness; and disposing a stabilizer in at least a portion of the carrier. The stabilizer is at least partially formed from a second elastomeric body material having a second hardness greater than the first hardness.

This summary is provided to introduce a selection of concepts in a simplified form that are further described below in the Detailed Description. This summary is not intended to identify key features or essential features of the claimed subject matter, nor is it intended to be used to limit the scope of the claimed subject matter.

### Brief Description of the Drawings

The same number represents the same element or same type of element in all drawings.
FIG. 1 illustrates a cut-away view of anatomy of an ear having a cochlear implant that can benefit from technologies disclosed herein.
FIG. 2, which is made up of FIGS. 2A-E, illustrates an example therapeutic element assembly in accordance with certain embodiments herein.
FIG. 2A illustrates a side view of the therapeutic element assembly in accordance with certain embodiments herein.
FIG. 2B illustrates a detail view of a portion of the therapeutic element assembly of FIG. 2A in accordance with certain embodiments herein.
FIG. 2C illustrates a cross-section view of a portion of the therapeutic element assembly of FIG. 2A taken along the line C-C in accordance with certain embodiments herein.
FIG. 2D illustrates a cross-section view of a portion of the therapeutic element assembly of FIG. 2A taken along the line D-D in accordance with certain embodiments herein.
FIG. 2E illustrates a cross-section view of a portion of the therapeutic element assembly of FIG. 2A taken along the line E-E in accordance with certain embodiments herein.
FIG. 3, which is made up of FIGS. 3A-3C, illustrates side views of different examples of a stabilizer in accordance with certain embodiments herein.
FIG. 3A illustrates a stabilizer having portions made from different materials having different characteristics along the stabilizer's length in accordance with certain embodiments herein.
FIG. 3B illustrates a stabilizer being tapered along the stabilizer's length in accordance with certain embodiments herein.
FIG. 3C illustrates a stabilizer having a stepped reduction along the stabilizer's length in accordance with certain embodiments herein.
FIG. 4 illustrates a configuration of a stabilizer having a concavity in accordance with certain embodiments herein.
FIG. 5 illustrates a side view of an example therapeutic element assembly having a stabilizer shown after insertion into a cochlea in accordance with certain embodiments herein.
FIG. 6 illustrates an example process for manufacturing a therapeutic element assembly having a stiffener in accordance with certain embodiments herein.

### Detailed Description

Examples disclosed herein include example apparatuses and methods for facilitating the temporary or permanent implantation of one or more therapeutic elements into a patient. For ease of understanding, many examples herein are described below in the context of cochlear implants with the therapeutic elements being electrodes. Cochlear implants use direct electrical stimulation of auditory nerve cells to bypass absent or defective hair cells that normally transduce acoustic vibrations into neural activity. The electrodes are inserted into the scala tympani of the cochlea so that the electrodes can differentially activate auditory neurons that normally encode differential pitches of sound. Such devices are also used to treat a smaller number of patients with bilateral degeneration of the auditory nerve. For such patients, the cochlear implant provides stimulation of the cochlear nucleus in the brainstem.

Examples herein can be used in conjunction with a cochlear implant, such as a CONTOUR, FREEDOM, NUCLEUS, or COCHLEAR implant sold by COCHLEAR LIMITED, Australia. Example cochlear implants are described in U.S. Patent Nos. 4,532,930; 6,537,200; 6,565,503; 6,575,894; and 6,697,674. It should be understood to those of ordinary skill in the art that examples disclosed herein can be used in other medical devices. Such medical devices can include, for example, prosthetic hearing implants, neurostimulators, cardiac pacemakers, cardiac defibrillators, sleep apnea management stimulators, seizure therapy stimulators, vestibular implants, and bionic eyes, as well as other medical devices that utilize an elongate carrier to temporarily or permanently implant, deliver or otherwise introduce a therapeutic element (e.g., an inert agent, a pharmacological agent, a sensor, a device, or an electrode) into a recipient.

In many examples, the flexibility of therapeutic element assemblies can beneficially minimize trauma to anatomical structures during insertion. But therapeutic element assemblies that are too flexible can be prone to buckling during insertion. For example, within the cochlear implant context, without sufficient stiffness the electrode assembly (the therapeutic element assembly of a cochlear implant) can be too soft and flexible to allow insertion to 360 degrees and beyond. Some approaches to having sufficiently flexible electrode assemblies include the use of tapering to progressively increase the cross section of the electrode assembly towards the basal end. Other approaches include the use of a stiffener embedded in the electrode assembly, which allows the cross section and volume (and therefore disturbance to anatomical structures and fluid pressure) of the electrode assembly to be reduced.

As a specific example, electrode assemblies of cochlear implants (e.g., the COCHLEAR NUCLEUS CI422, COCHLEAR NUCLEUS CI522, or COCHLEAR NUCLEUS HYBRID L) can incorporate a basal platinum stiffening member. Such stiffeners are tapered and annealed to minimize sudden changes in stiffness along the length of the array. However the relative stiffness of platinum compared to the silicone of the electrode assembly is high compared to metal stiffeners, disclosed examples can provide better control over the distribution of stiffness along the electrode assembly and have improved durability due to the tendency of example stabilizers herein to elastically (rather than plastically) deform.

Examples disclosed herein include the use of an elastomeric region (e.g., made of silicone) having a greater hardness than the silicone of the therapeutic element assembly to provide stiffness. The stabilizer can be configured to provide stiffness to the therapeutic element assembly while having a smaller change in relative stiffness with the rest of the assembly compared to traditional stiffeners. The elastomeric region can be referred to as a stabilizer and can have one or more of any of a variety of characteristics. For example, the stabilizer can be made of a single grade or multiple grades of elastomers of increasing durometer from the distal to proximal end of the stabilizer. The stabilizer can be separately molded and then encapsulated in the therapeutic element assembly during a final molding process. The stabilizer can be formed by molding the therapeutic element assembly with a lumen or internal hollow space that is post-filled with liquid elastomer (e.g., silicone) then cured. The stabilizer can be tapered to provide smooth grading of stiffness. The stabilizer can have features such as holes or grooves to promote bending at desired locations (e.g., to facilitate insertion). The stabilizer can have holes or other features to provide positive mechanical integration with the body material (e.g., silicone) of the carrier of the therapeutic element assembly. The stabilizer can be continuous with a handle. The stabilizer can itself be stiffened by a metallic or other element embedded in its proximal region (e.g., outside the cochlea). For example, a metallic stiffener can be disposed within the stabilizer without extending distally past the collar. Such a stiffener can provide further stiffness and stabilization with the handle. This metallic element may extend into the lead to produce a malleable lead to prevent springing during fixation. The stabilizer can be molded with bumps or other protrusions to center the stabilizer within a molding die while still being largely encapsulated by the carrier. Where the stabilizer is used with cochlear implants, the stabilizer can continue basally outside the intracochlear region to provide stability and prevent buckling/hinging outside the cochlea.

Beneficially, the stabilizer can tune the bending stiffness of the carrier of the therapeutic element assembly to vary along the length of the carrier without points of substantial discontinuity in stiffness. With metallic stiffeners, due to the very large difference in material properties between even the softest metal and the elastomer material of the therapeutic element assembly, there can be a step change in bending stiffness at the end of the metallic stiffener. By contrast, with a stabilizer that is made of similar material to the surrounding material (e.g., the material of the therapeutic element assembly that surrounds the stiffener), it is possible to more gradually vary the stiffness along the length of the therapeutic element assembly. For instance the stabilizer can be made from the same material as the body of the therapeutic element assembly but with increased hardness (e.g., both can be made from silicone, but the stabilizer can be made from a harder silicone).

The elastomeric stabilizer element can further advantageously elastically deform if bent. By contrast, a metallic stiffener tends to plastically deform if accidentally bent or buckled during manufacturing or surgery (e.g., before or during insertion). While the therapeutic element assembly can be manually re-straightened, the points at which the stiffener bent would retain residual stress, and act as weak points at which buckling is likely to occur during insertion. By contrast, the elastomeric stabilizers disclosed herein can be configured to elastically deform if accidentally bent or buckled during manufacturing or in surgery. While the electrode wires within the array (e.g., which may be made of platinum or an alloy) may still kink during bending, the presence of the elastomeric stiffener tends to support the array at these locations and minimizes risk of repeat buckling, and also produce a smoother, more distributed pattern of contact pressure with the lateral wall during insertion.

A further advantage is in manufacturability. An elastomeric stabilizer can be molded to almost any shape very with high repeatability. By contrast, a metal stiffener, which must typically be tapered in order to minimize sudden changes in stiffness, can be relatively difficult to manufacture due to the tight tolerances required. Geometry is generally also constrained by manufacturing considerations. For example, a metallic element may be tapered in one direction by a forming or grinding process, however tapering in a second plane requires a second processing step which adds cost and complexity. The material generally used for the stiffener is platinum, due to its biocompatibility and malleability, making the stiffener a significant factor in the overall cost of the electrode. By contrast, the stabilizers disclosed herein can be formed from an elastomer in a single forming process (e.g., as compared to the multiple processing steps required to form a metallic stiffener). Further, where the stabilizer is formed from an elastomer, the stabilizer 208 is non-conductive, so the carrier can be manufactured without the need for insulation to prevent contact between the wires running through the carrier and a metallic stiffener.

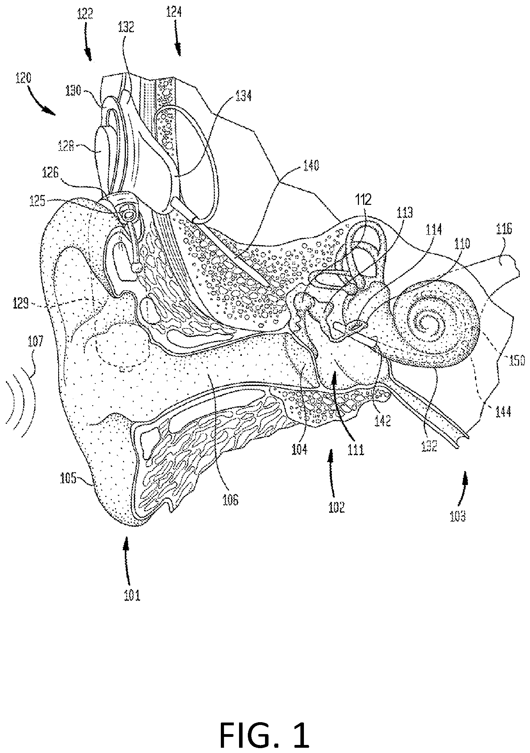

An example medical device that can benefit from the stabilizer technology disclosed herein is shown in FIG. 1.

### Example Medical Device

Medical devices can benefit from stabilizers disclosed herein, particularly medical devices having a therapeutic element assembly that is inserted into a target region of a recipient. For example, stabilizers disclosed herein can facilitate insertion of an electrode array into a cochlea of a recipient.

FIG. 1 illustrates a cochlear implant and a cut-away view of the relevant components of an outer ear 101, a middle ear 102, and an inner ear 103. In a fully functional ear, the outer ear 101 comprises an auricle 105 and an ear canal 106. The auricle collects acoustic waves 107 and channels the acoustic waves into and through the ear canal 106. Disposed across the distal end of the ear canal 106 is a tympanic membrane 104 that vibrates in response to the acoustic waves 107. This vibration is coupled to the oval window 110 through the ossicles 111 of the middle ear 102. The ossicles 111 are bones of the middle ear 102 and include the malleus 112, the incus 113 and the stapes 114. The ossicles 111 serve to filter and amplify the acoustic waves 107 and to cause the oval window 110 to vibrate. Such vibration sets up waves of fluid motion within the cochlea 132. Such fluid motion, in turn, activates tiny hair cells (not shown) that line the inside of the cochlea 132. Activation of the hair cells causes appropriate nerve impulses to be transferred through the spiral ganglion cells and the auditory nerve 116 to the brain (not shown), where they are perceived as sound. In people experiencing sensorineural hearing loss, there is an absence or destruction of the hair cells. A cochlear implant can be used to directly stimulate the spinal ganglion cells to provide a hearing sensation to such people.

FIG. 1 further shows how the cochlear implant 120 is positioned in relation to the outer ear 101, the middle ear 102, and the inner ear 103. The cochlear implant 120 has an external assembly 122 that is directly or indirectly attached to the body of the recipient, and an internal component assembly 124 that is temporarily or permanently implanted in the recipient. The external assembly 122 has a microphone 125 for detecting sound that is outputted to a behind-the-ear speech processing unit 126. During use, the microphone 125 can be worn on the recipient's pinna or another suitable location, such as a lapel of the recipient's clothing. The speech processing unit 126 can generate coded signals that are provided to an external transmitter unit 128, along with power from a power source such as a battery.

The external transmitter unit 128 includes an external coil 130 and, preferably, a magnet (not shown) secured directly or indirectly in the external coil 130. The internal components include an internal receiver/transmitter unit having an internal coil (not shown) that receives and transmits power and coded signals from the external assembly 122 to a stimulator 134 to apply the coded signal along a therapeutic element assembly 140. The therapeutic element assembly 140 enters the cochlea 132 at a cochleostomy region 142 and has one or more of the electrodes 150 positioned to be substantially aligned with tonotopically-mapped portions of the cochlea 132. Signals generated by the stimulator 134 are applied by the electrodes 150 of the electrode array 144 to the cochlea 132, thereby stimulating the auditory nerve 116. It should be appreciated that although in the embodiment shown in FIG. 1 electrodes 150 are arranged in an electrode array 144, other arrangements are possible.

The therapeutic element assembly 140 can be configured to assume an optimal electrode position in the cochlea 132 upon or immediately following implantation into the cochlea 132. It is also desirable that the therapeutic element assembly 140 be configured such that the insertion process causes minimal trauma to the sensitive structures of the cochlea 132. Usually a therapeutic element assembly is held in a straight configuration at least during the initial stages of the insertion procedure, then conforming to the natural shape of the cochlea during and subsequent to implantation.

While cochlear implant system 100 is described as having external components, in another embodiment, one or more components can be implantable. In such embodiments, a controller can be contained in a hermetically sealed housing or the housing of the stimulator 134.

While FIG. 1 illustrates a cochlear implant 120 that can benefit from technologies disclosed herein, other devices and systems can benefit from disclosed technologies. For instance, the technology can be used in conjunction with any apparatuses having a flexible elongate carrier configured to introduce a therapeutic element into a recipient. For instance, disclosed technology can be used to insert therapeutic elements of any of a variety of sensory prostheses. For example, the sensory prosthesis can be a prosthesis relating to one or more of the five traditional senses (vision, hearing, touch, taste, and smell) and/or one or more of the additional senses. As described above, a sensory prosthesis can be an auditory prosthesis medical device (e.g., a cochlear implant 120) configured to treat a hearing-impairment of the recipient. Where the sensory prosthesis is an auditory prosthesis, the sensory prosthesis can take a variety of forms including a cochlear implant, an electroacoustic device, a middle ear device, a totally-implantable auditory device, a mostly-implantable auditory device, an auditory brainstem implant device, other auditory prostheses, and combinations of the foregoing (e.g., binaural systems that include a prosthesis for a first ear of a recipient and a prosthesis of a same or different type for the second ear). In examples, the sensory prosthesis can be or include features relating to bionic eyes. Technology disclosed herein can also be relevant to applications with devices and systems used in for example, sleep apnea management, tinnitus management, and seizure therapy. Technology disclosed herein can be used with sensory devices such as consumer auditory devices (e.g., a hearing aid or a personal sound amplification product. Generally, disclosed examples replace or supplement one or more components of a therapeutic element assembly.

### Therapeutic element assembly

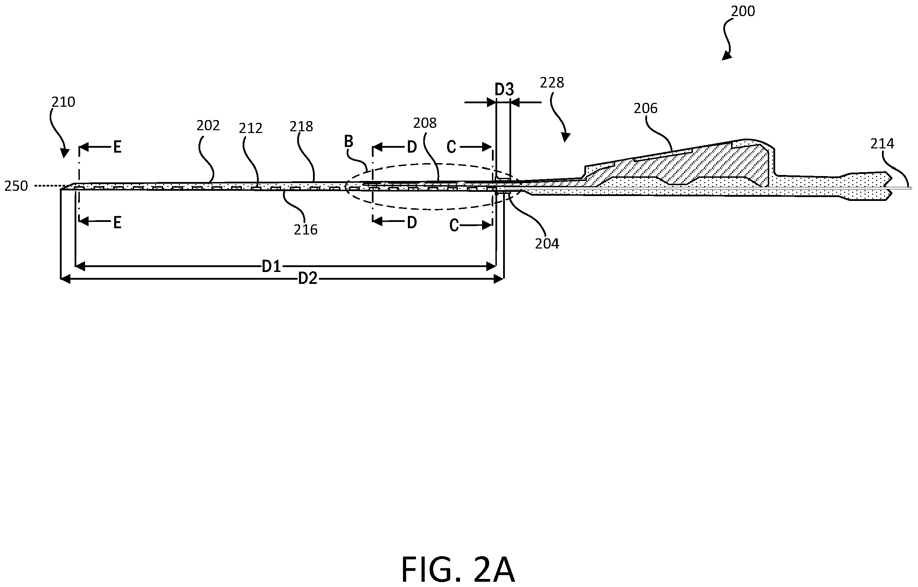

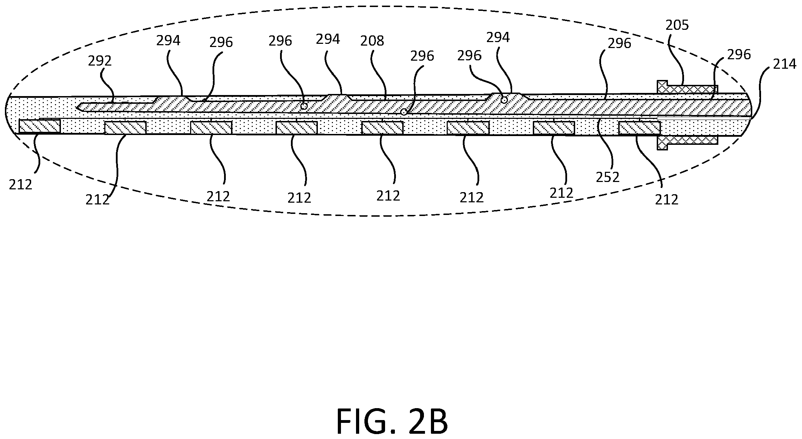

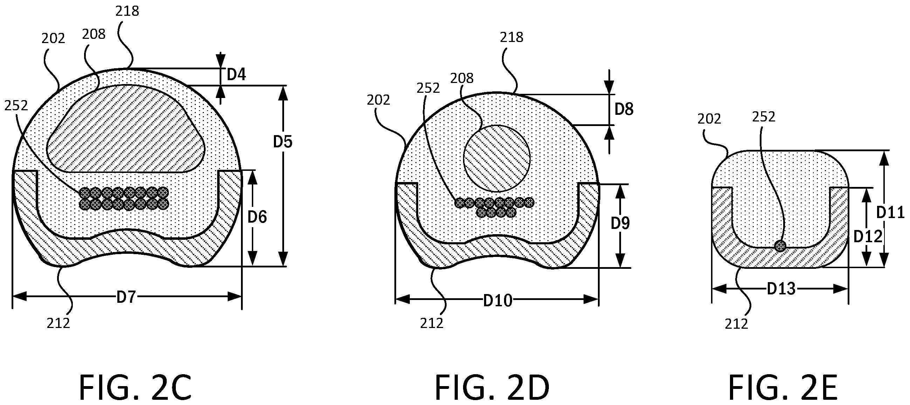

FIG. 2 is made up of FIGS. 2A-E. FIG. 2A illustrates a side view of an example therapeutic element assembly 200 in accordance with certain embodiments of the invention. FIG. 2B illustrates a detail view of a portion of the therapeutic element assembly 200 of FIG. 2A. FIG. 2C illustrates a cross-section view of a portion of the therapeutic element assembly 200 of FIG. 2A taken along the line C-C. FIG. 2C illustrates a cross-section view of a portion of the therapeutic element assembly 200 at or proximate a most-proximal therapeutic element 212. FIG. 2D illustrates a cross-section view of a portion of the therapeutic element assembly 200 of FIG. 2A taken along the line D-D. FIG. 2D illustrates a cross-section view of a portion of the therapeutic element assembly 200 at or proximate a distal-most therapeutic element 212 before the distal end of the stabilizer 208 (e.g., the distal-most section of the therapeutic element assembly 200 having both a therapeutic element 212 and the stabilizer 208). FIG. 2E illustrates a cross-section view of the therapeutic element assembly 200 of FIG. 2A taken along the line E-E. FIG. 2E illustrates a cross-section view of a portion of the therapeutic element assembly 200 at or proximate a distal-most therapeutic element 212 of the therapeutic element assembly 200.

The therapeutic element assembly 200 includes a carrier 202. The carrier 202 can be the portion of the therapeutic element assembly 200 that holds the therapeutic elements 212. The carrier 202 can be configured to be inserted into a treatment site and appropriately position the therapeutic elements 212 proximate a region to be treated. The carrier 202 has a distal region 210 and a proximal region 228 connected to the collar 204. In some examples, the therapeutic element assembly includes a collar 204, a handle 206, and a lead 214. The proximal end of collar 204 is connected to the handle 206.

It should be understood that the terms medial surface, medial direction and the like are generally used herein to refer to the surfaces, features and directions toward a treatment site (e.g., toward the center of a cochlea), while the terms lateral surface, lateral direction and the like are generally used herein to refer to surfaces, features and directions away from the treatment site (e.g., toward the exterior of the cochlea). For example, where the therapeutic element assembly 200 is for a cochlear implant, the longitudinally-extending surface of the carrier 202 that faces the interior of cochlea 132 when implanted can be referred to as a medial surface 216 of the carrier 202. The opposing side of the carrier 202 that faces the external wall and bony capsule of cochlea 132 when implanted can be referred to as a lateral surface 218.

A plurality of spaced-apart therapeutic elements 212 are mounted on or in the carrier 202. For ease of understanding, the therapeutic elements 212 are referred to herein as electrodes 212, but, as discussed above, any of a variety of one or more therapeutic elements can be used instead of or in addition to electrodes. The electrodes 212 can be disposed in a linear or non-linear array on or in the carrier 202, and may be positioned to align with predetermined tonotopically-mapped regions of the cochlea 132. In one alternative embodiment, the electrodes 212 have variable spacing as described in U.S. Patent No. 7,881,811, which is titled "Flexible Electrode Assembly Having Variable Pitch Electrodes".
Such arrangements allow for individual electrodes 212 to be energized to stimulate selected regions of the cochlea 132.

In one example, the electrodes 212 are half-band electrodes disposed on the medial surface 216 of the carrier 202. It should be appreciated, however, that any electrodes 212 now or later developed suitable for a particular application or therapeutic objective may be used in alternative embodiments. For example, in one alternative embodiment, the electrodes 212 are banded electrodes extending substantially around the carrier 202. In another alternative embodiment, the electrodes 212 do not laterally extend to or around the edges of the carrier 202.

In many examples, each of the electrodes 212 is arranged orthogonal to a longitudinal axis 250 of the carrier 202. But other relative positions and orientations may be implemented in alternative embodiments. Further, the quantity of the electrodes 212 can vary from as few as one electrode to as many as twenty-four or more electrodes. In some examples, at least one of the electrodes 212 has a surface that is at least adjacent the medial surface 216 of the carrier 202. One or more of the electrodes 212 can have a surface that is co-located with the medial surface 216 of the carrier 202. In another example, the surfaces of the electrodes 212 are raised above or recessed into the medial surface 216 of the carrier 202. The electrodes 212 can be manufactured from a biocompatible conductive material such as platinum, but other materials or combinations of materials can be used. In other examples, the electrodes 212 can be coated with a biocompatible covering that does not substantially interfere with the transfer of the stimulation signals to the cochlea 132.

As can be seen in FIG. 2D, each electrode 212 can be electrically-connected to at least one wire 252. Each wire 252 can be a multi- or single-filament wire that is embedded within the flexible carrier 202, collar 204, handle 206, and lead 214. The wires 252 are embedded in the volumetric core of carrier 202. In collar 204, the stabilizer 208 and the wires 252 extend or travel through a central volumetric core. In an alternative example, the wires 252 can be located at or near the medial surface 216 or the lateral surface 218 of the carrier 202. In other embodiments, the wires 252 are embedded in different regions of the carrier 202 to facilitate attainment of a desired curvature, to maintain orientation of the carrier 202 once the carrier 202 is implanted, to attain a desired level of isolation between the stabilizer 208 and the wires 252, to achieve other objectives, or combinations thereof. The stimulator 134 can provide electrical stimuli to the electrodes 212 via the wires 252. In one embodiment, the wires 252 are connected to the electrodes 212 by welding or another suitable connecting technique.

The number of wires 252 connected to each of the electrodes 212 may vary.
For example, in one example, at least two electrically conducting wires 252 are connected to each of one or more electrodes 212. It should also be appreciated that suitable transmission means other than filament wires may be used to communicably couple the stimulator 134 and the electrodes 212.

In the illustrated example, a lead 214 longitudinally extends through the carrier 202, collar 204 and the handle 206 to electrically connect the electrodes 212 with a device, such as the stimulator 134 of FIG. 1. In an example, the lead 214 can be about 80 mm long. The lead 214 can include a bundle of wires running from the electrodes.

The stimulator 134 can be encased within a housing that is implantable within the recipient. Where the stimulator 134 is for a cochlear implant, the housing can be implantable within a recess in bone behind the ear posterior to the mastoid. In one example, the lead 214 extends from the handle 206 to the stimulator 134 (or the housing of stimulator 134). In one particular embodiment, the lead 214 is continuous (e.g., with no electrical connectors required to electrically connect the therapeutic element assembly 200 to the stimulator 134). One advantage of this arrangement is that there is no requirement for a surgeon implanting the therapeutic element assembly 200 to make the necessary electrical connection between the wires 252 extending from the electrodes 212 and the stimulator 134.

The handle 206 is a portion by which the surgeon implanting the therapeutic element assembly 200 can grasp and manipulate the therapeutic element assembly 200. In some examples, the handle 206 provides for improved handling and the ability to identify electrode orientation. In some examples, the handle 206 can be configured as described in U.S. Patent. No. 7,349,744. The stabilizer 208 can be disposed in the handle, which can ease the manufacturing process and reduce or eliminate the need for an additional stiffener for the handle to be constructed and added. The inside of the handle 206 can have features to improve flow of material used to form the stabilizer 208 during manufacture. For example, the features can include one or more wings, bumps, ridges, channels, other features, or combinations thereof configured to enhance or inhibit the flow of material during manufacture.

In some examples, the distal region 210 of the carrier 202 is profiled. The profile can help guide the carrier 202 during the insertion process, such as by reducing friction. Alternative embodiments of the distal region 210 are described in U.S. Patent No. 7,881,811. In other examples, the distal region 210 can be as described in U.S. Patent No. 7,962,226.

In some examples, the therapeutic element assembly can include a collar 204. The collar 204 can serve as both a region for grasping the therapeutic element assembly 200 and also act to prevent insertion of the carrier 202 beyond a predetermined maximum depth to reduce the risk of the surgeon over-inserting the therapeutic element assembly 200, which could otherwise cause trauma to anatomical structures. In certain examples, the predetermined maximum depth is as described in the above-referenced applications or in U.S. Patent Nos. 7,881,811; 7,962,226; and 8,630,721. The collar 204 is described in further detail in the above applications.

As illustrated, the carrier 202 includes a stabilizer 208. The stabilizer 208 can be permanently embedded in at least the proximal region 228 of the carrier 202. In such examples, the stabilizer 208 cannot be removed from the carrier 202 without damaging one or both of the carrier 202 or the stabilizer 208. In the illustrated example in FIGS. 2A and 2B, the stabilizer 208 can extend from an external portion (e.g., an extracochlear region) of the therapeutic element assembly 200 through the collar 204 and into the carrier 202. In alternative embodiments, the stabilizer 208 need not be embedded in the collar 204, and the stabilizer 208 can longitudinally extend further through carrier 202 to terminate at any desired location along the length of carrier 202. Where the therapeutic element assembly 200 is configured for use with a cochlear implant, the stabilizer 208 can extend into the carrier 202 such that the stabilizer 208 terminates just before the lateral wall of the first turn of the cochlea 132 when the carrier 202 is completely inserted into the cochlea 132.

The stabilizer 208 can be configured to increase the stiffness of the carrier 202 in the regions in which stabilizer 208 is located. As such, stabilizer 208 assists in the prevention of buckling or deformation of the carrier 202 in such regions during insertion of the carrier 202 into the cochlea 132. In particular, the stabilizer 208 assists in maintaining the proximal region 228 of carrier 202 in a sufficiently-straight configuration when subjected to the forces typically experienced during implantation. This allows the carrier 202 and the electrodes 212 to be fully implanted into cochlea 132 without being subject to insertion forces that may damage the delicate structures of the cochlea.

Additionally, the stabilizer 208 can be configured to cause the electrodes 212 to be positioned closer to a treatment site (e.g., the inner wall of the cochlea 132) because a straight carrier 202 may generally take a more lateral position (e.g., in the basal region of the cochlea 132). As a result, the distance from the stimulating surface of carrier 202 to treatment site (e.g., the auditory nerve endings proximate the treatment site) is substantially less than would be the case if the stabilizer 208 were not embedded in the therapeutic element assembly 200. The stabilizer 208 can provide similar benefits to cochlear implants in the basal region as a perimodiolar electrode (e.g., the perimodiolar electrode described in U.S. Patent No. 6,421,569). While many examples herein describe the material of the stabilizer 208 as having a stiffness greater than that of the material of the carrier 202, It should also be appreciated that the stiffness of the material of the stabilizer 208 may be less than, the same as, or greater than the stiffness of the carrier 202, so long as the presence of stabilizer 208 in regions of carrier 202 results in at least one of such regions having a reduced likelihood of deformation.

The stabilizer 208 can be formed from or otherwise comprise an elastomeric material. The elastomeric material can be a medical grade elastomeric material. The elastomeric material can be a silicone elastomer. In an example, the silicone elastomer has a hardness of 80 Shore A hardness units. For instance, the silicone elastomer can be made from MED-4480 silicone rubber produced by NUSIL TECHNOLOGY LLC. The silicone elastomer can have a tensile strength of 1030 PSI, an elongation of 265%, and a tear resistance of 90 PPI.

The stabilizer 208 can be constructed from an elastomeric body material that is different from the elastomeric body material of the carrier 202. For example, the carrier 202 can be manufactured using a first elastomeric body material having a first hardness. The stabilizer 208 can extend through at least a portion of the first elastomeric body material and be manufactured using a second elastomeric body material. The second elastomeric body material can have a second hardness greater than the first hardness. In an example, the stabilizer 208 can be formed from a material having a hardness that is 10%, 15%, 20%, 25%, 30%, 35%, 40%, 45%, 50%, 55%, 60%, 65%, 70%, 75%, 80%, 85%, 90%, 95%, or 100% harder than the material from which the carrier 202 is constructed. In another example, the difference in hardness between material of the stabilizer 208 and the material of the carrier 202 can be less than 10%, 15%, 20%, 25%, 30%, 35%, 40%, 45%, 50%, 55%, 60%, 65%, 70%, 75%, 80%, 85%, 90%, 95%, or 100% of the hardness of the material from which the carrier 202 is constructed. As a specific example, the carrier 202 can be constructed from a silicone elastomer having a hardness of 60 Shore A hardness units (e.g., MED-4860 silicone rubber produced by NUSIL TECHNOLOGY LLC). In such an example, the stabilizer 208 can be constructed from a silicone elastomer having a hardness of 80 Shore A hardness units. In such an example, the material from which the stabilizer 208 is constructed has a hardness that is approximately one-third greater than the hardness of the carrier 202 (i.e., 20 Shore A hardness units harder). In another example, the hardness of the carrier 202 is less than or equal to 60 durometer type A hardness, and the hardness of the stabilizer 208 is greater than the first hardness and less than or equal to 80 durometer type A hardness.

The stabilizer 208 can be configured to variably decrease the flexibility of one or more regions of the carrier 202 (e.g., the proximal region 228). For example, the stabilizer 208 can have a tapered profile, thereby variably decreasing the flexibility of the proximal region 228 of

The stabilizer 208 can include or define features to promote or resist certain behavior. For instance, the stabilizer 208 can define one or more flex structures 292 configured to promote bending of the stabilizer 208 in predetermined locations. For instance, the one or more flex structures 292 can include one or more holes, grooves, or other areas of relatively less material. The stabilizer 208 can define one or more integration structures 294 configured to provide positive mechanical integration of the stabilizer 208 with the flexible elongate carrier 202. The stabilizer 208 can facilitate resisting the stabilizer 208 and the carrier 202 separating (e.g., peeling apart). The stabilizer 208 can define or include one or more protrusions 296 to facilitate centering the stabilizer 208 within a molding die for the carrier 202. The protrusions 296 can be bumps, cylindrical protrusions, rectangular protrusions, or other kinds of protrusions. The protrusions contact the die cavity and keep the main body of the stabilizer 208 within the rest of the carrier 202.

The stabilizer 208 can take up a percentage of the area of a portion of the carrier 202 in cross section perpendicular to the long axis of the carrier 202 that is at least x%, where x is an integer in the range between 1 and 90 in increments of one. In an example, the percentage of the area of a portion of the stabilizer in cross section perpendicular to the long axis of the carrier 202 that is at least 10%, at least 20%, at least 30%, at least 40%, at least 50%, at least 60%, at least 70%, at least 80%, or at least 90%. The area of the portion of the stabilizer mentioned above regarding the area of the portion of the stabilizer in cross section can be located at a region located at *y*% of the way along the way along the length of the carrier (measured from the distal end of the carrier), where x is an integer in the range between 1 and 90 in increments of one. In an example, the area is located at a point 10%, 20%, 30%, 40%, 50%, 60%, 70%, 80%, or 90% of the way along the carrier 202. The stabilizer can reinforce the handle and continuously stiffens the carrier 202. This improves the control offered to the user. The elastic stiffener is less likely to permanently deform. For instance, the device may accidentally deform, bend, or buckle during handling or insertion. Where a metal or glass stiffener may suffer from plastic deformation from such deformation, bending, or buckling, an elastomeric stiffener may elastically deform, which is beneficial.

In some examples, the carrier 202 can further include a metallic stiffener. The metallic stiffener can be disposed in one or both of the material of the carrier 202 and the material of the stabilizer 208. In some examples, the stabilizer 208 is configured to bridge a flexibility gap between the material of the carrier 202 and a distal portion of the metallic stiffener. In some examples, the metallic stiffener is configured to provide increased proximal stiffness compared to the use of the stabilizer 208 alone. In some examples, the metallic stiffener is disposed in the handle 206 and extends distally and stops proximate the collar 205. In other examples (e.g., examples without the collar 205), the metallic stiffener extends distally and stops proximate the most-proximal electrode 212.

FIGS. 2A-2E illustrate various example distances between points of the therapeutic element assembly 200. FIG. 2A shows distances *D1-D3.* Distance *D1* is the distance from the beginning of a first electrode 212 (e.g., the proximal-most electrode 212) to an end of a last electrode 212 (e.g., the distal-most electrode 212). Distance *D2* is the distance from the distal end of the collar 204 to the distal tip of the carrier 202. Distance *D3* is the length of the collar 204.

FIG. 2C shows distances *D4-D7.* Distance *D4* is the distance from the lateral surface 218 to the top of the stabilizer 208 for the portion of the therapeutic element assembly 200 shown in cross-section in FIG. 2C, and distance *D5* is the distance from the top of the stabilizer 208 to a bottom of the carrier 202 for the portion of the therapeutic element assembly 200 shown in cross-section in FIG. 2C, such that the sum of distance *D4* and distance *D5* is the height of the carrier 202 for the illustrated slice. Distance *D6* is the distance from the bottom of the carrier 202 to the top of the electrode 212 for the portion of the therapeutic element assembly 200 shown in cross-section in FIG. 2C. Distance *D7* is width of the carrier 202 for the portion of the therapeutic element assembly 200 shown in cross-section in FIG. 2C.

FIG 2D illustrates distances *D8-D10.* Distance *D8* is the distance from the lateral surface 218 to the top of the stabilizer 208 for the portion of the therapeutic element assembly 200 shown in cross-section in FIG. 2D. Distance *D9* is the distance from the bottom of the carrier 202 to the top of the electrode 212 for the portion of the therapeutic element assembly 200 shown in cross-section in FIG. 2D. Distance D10 is a width of the carrier 202 for the portion of the therapeutic element assembly 200 shown in cross-section in FIG. 2D.

FIG. E illustrates distances D11-D13. Distance *D11* is the height of the carrier 202 for the portion of the therapeutic element assembly 200 shown in cross-section in FIG. 2E. Distance *D12* is the distance from the bottom of the carrier 202 to the top of the electrode 212 for the portion of the therapeutic element assembly 200 shown in cross-section in FIG. 2E. Distance *D13* is the width of the carrier 202 for the portion of the therapeutic element assembly 200 shown in cross-section in FIG. 2E.

Table I, below, illustrates example measurements in millimeters for the distances where the therapeutic element assembly 200 is used in conjunction with a cochlear implant. In examples, one or more of the distances can vary by ±0.1 or ±0.2. Other measurements can be used.

| **TABLE I** | |
|---|---|
| Distance | Length (mm) |
| *D1* | 19.10 |
| *D2* | 20.00 |
| *D3* | 0.50 |
| *D4* | 0.05 |
| *D5* | 0.45 |
| *D6* | 0.20 |
| *D7* | 0.60 |
| *D8* | 0.08 |
| *D9* | 0.20 |
| *D10* | 0.52 |
| *D11* | 0.25 |
| *D12* | 0.20 |
| *D13* | 0.35 |

As can be seen by comparing FIGS. 2C and 2D, the size and shape of the stabilizer 208 can vary along the length of the stabilizer 208. The stabilizer 208 can transition from a first shape to a second shape along at least a portion of the length of the stabilizer 208. In an example, the first shape is the shape of stabilizer 208 shown in FIG. 2C in cross section having a trapezoidal shape with rounded corners, and the second shape is the shape of the stabilizer 208 shown in FIG. 2D in cross section having a substantially circular shape. In another example, the first shape is a non-circular ellipse and the second shape has a circular shape. In an example, the first shape has a width greater than the second shape. In another example, the first shape has a height greater than the second shape.

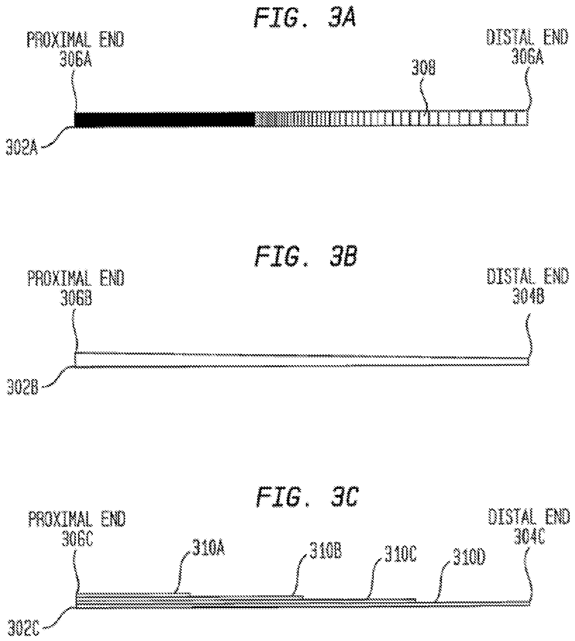

FIGS. 3A through 3C are side views of different example of the stabilizer 208, referred to herein as stabilizers 302A, 302B, and 302C, respectively (generally and collectively referred to as stabilizers 302). The stabilizers 302 are configured to be embedded in examples of therapeutic element assemblies 200 described above. In these examples, the stiffness or malleability of stabilizer 208 is longitudinally varied such that, for example, the distal portion 230 of the carrier 202 is more flexible than the proximal portion of the carrier 202. Such variability may be attained, for example, by using materials having different characteristics (e.g., as shown in FIG. 3A), tapering (e.g., as shown in FIG. 3B), or stepped reduction (e.g., as shown in FIG. 3C). In these and other examples, there preferably is a gradual transition from the more flexible distal end 304 to the stiffer proximal end 306 of the carrier 202. It should be appreciated, however, that such a gradual transition in the noted direction may be particular to the example application of cochlear implants and may vary differently in other applications.

Referring to FIG. 3A, stabilizer 302A is formed from a variety of materials having differing stiffness. In the embodiment shown in FIG. 3A, longitudinally-adjacent regions 308 (only one is identified for simplicity) of the stabilizer 302A are made from different materials having different qualities (e.g., different hardness) or that were subject to different curing processes resulting in regions 308 having different hardness or other qualities. In particular, longitudinally successive regions 308 have incrementally greater or less flexibility are depicted in FIG. 3A by successively increasing and decreasing widths of regions 308. In an example, the regions 308 can be manufactured via a series of injection molding steps where grades of soft silicone are added and gaps are filled with harder silicone in such a way that there is a smooth variation in total stiffness.

Referring to FIG. 3B, the stabilizer 302B is, in this illustrated example, a unitary component that is tapered from a proximal end 306B toward a distal end 304B. The reduced volume of material along successive regions of stabilizer 302 results in a successively decreasing stiffness. It should be appreciated that the rate of taper will dictate the rate of change in flexibility of the carrier 202.

Referring to FIG. 3C, the stabilizer 302C is an integrated element having multiple elongate strips 310A-310D of differing lengths. The strips 310 can be formed of the same or different material, and may be manufactured to have the same or different stiffness. The strips 310 may be secured in any of a variety of manners. As shown in FIG. 3C, the stabilizer 302C has a stepped configuration, due to the different lengths of strips 310. As such, the stiffness provided by stabilizer 302C varies due to the cumulative contribution of each strip 310, which varies along its length. The strips 310 need not be arranged to form a continuous series of steps. For example, the desired flexibility of carrier 202 does not vary continuously, strips 310 may be configured such that, for example, the strip 310B is longer than the strip 3 IOC.

In addition to the embodiments illustrated in FIGS. 3A-3C, the variable stiffness can be achieved by utilizing any number of the following alone or in combination with each other or the embodiments described above: a plurality of stabilizer components spaced at various pitches to provide a variable stiffness; use of different materials at various intervals along the length of the stabilizer 208; varying dimensions of the stabilizer 208 or its component elements, etc. It should also be appreciated that the stabilizer 208 can be of any manufacturable cross-section, including round, square, rectangular, oval etc., and use any manufacturable method to provide variable stiffness along its length.

In alternative embodiments, the stabilizer 208 extends further into the carrier 202, providing regions of enhanced stiffness where desired. It should be appreciated that the regions of stiffness in the embodiments illustrated in FIGS. 3A-3C, or otherwise, need not vary regularly or consistently.

This stiffening arrangement may be similar to that described in US 8,812,121.

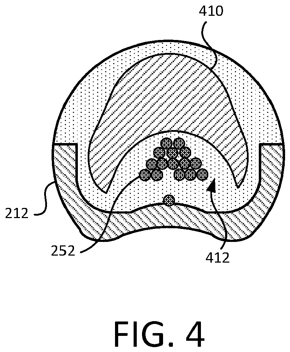

FIG. 4 illustrates a configuration of the stabilizer 410 having a concavity 412. As illustrated, at least one of the wires 252 is embedded within the body material of the therapeutic element assembly 200 and within the concavity 412 along at least a portion of the length of the at least one of the wires 252. Advantageously, this arrangement allows for a compact cross section of the carrier 202 while achieving stiffness and stabilization via the stabilizer 208. In examples, the wires 252 are arranged into a shape to facilitate fitting within the concavity 412. In the illustrated configuration, the wires 252 form a triangular shape configured to fit within the concavity 412. As a particular example, a flexible elongate carrier 202 can include a plurality of electrodes 212. Each respective electrode 212 can have a wire 252 extending therefrom for electrically connecting the respective electrode 212 to a device (e.g., an implantable stimulator device separate from the therapeutic element assembly 200). At a point along the flexible elongate carrier 202, the stabilizer 208 has a profile defining a concavity 412. At least one of the wires 252 is embedded within the first elastomeric body material and within the concavity 412.

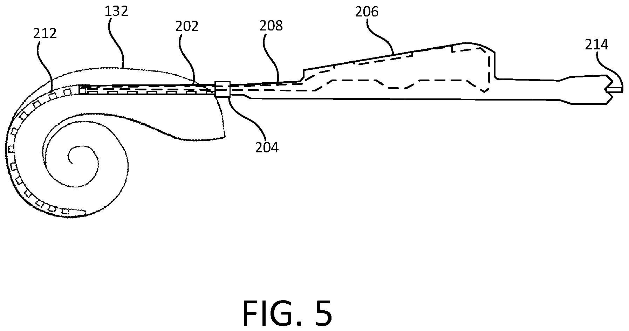

FIG. 5 is a side view of therapeutic element assembly 200 shown after insertion into a cochlea. As illustrated, the distance that the stabilizer 208 extends into the carrier 202 is such that the stabilizer 208 terminates just before a lateral wall of the first turn of cochlea 132 when the carrier 202 is completely inserted into cochlea 132. Advantageously, the stabilizer 208 can be configured to provide the carrier 202 with sufficient stiffness to allow the carrier 202 to be effectively inserted into cochlea 132, particularly once the carrier 202 encounters some resistance beyond the first turn of the cochlea 132. A further advantage of the variation in stiffness is to ensure that therapeutic element assembly 200 is suitable for various cochlea sizes. Cochlea sizes, and therefore the basal length, from the round window to the lateral wall of cochlea 132, vary slightly between recipients. The basal length is generally a straight path and is usually in the order of approximately 4 mm to 7 mm. The more flexible distal end of the stabilizer 208 ensures that the distal tip of the stabilizer 208 does not impact with the fragile structures of the cochlea. Rather, the distal end deforms allowing carrier 202 to curve whilst still ensuring the proximal region of the therapeutic element assembly 200 does not buckle or deform. Preferably, the variable stiffness also ensures that the carrier 202 forms a gradual curve rather than a sharp bend that could result by having a sudden change in mechanical stiffness.

### Manufacturing

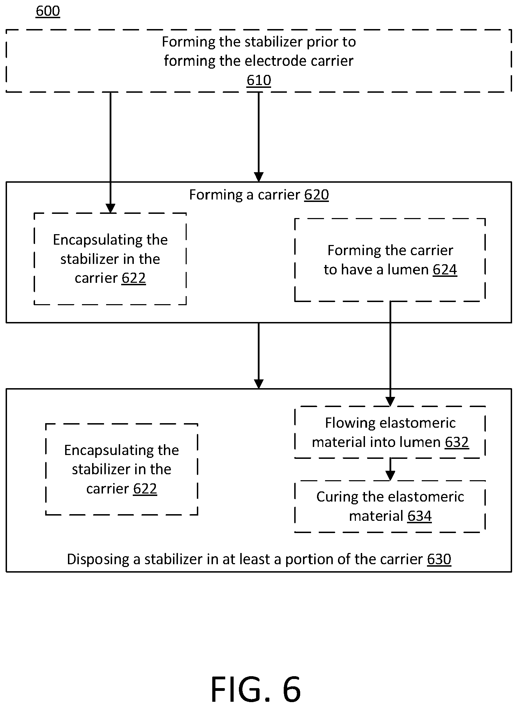

FIG. 6 illustrates an example process 600 for manufacturing the carrier 202 and associated components. As illustrated, the process 600 can begin with operation 610 or operation 620.

Operation 610 includes forming the stabilizer 208 prior to forming the carrier 202. The operation 610 can include, for example forming the stabilizer 208 using an injection molding process or another suitable manufacturing technique. During this operation 610, the stabilizer 208 can be at least partially formed from an elastomeric body material having a hardness greater than a hardness of a material from which the carrier 202 will be formed. Further, this operation 610 can include forming the stabilizer 208 with one or more protrusions 296 to facilitate centering the stabilizer 208 within a molding die in which the carrier 202 is formed. Following operation 610, the flow can move to operation 620.

Operation 620 includes forming the carrier 202 at least partially from a first elastomeric body material having a first hardness. The carrier 202 can be formed using injection molding or another suitable manufacturing technique.

In some examples, operation 620 includes operation 622. Operation 622 includes encapsulating the stabilizer 208 (e.g., as formed in operation 610) in the carrier 202. The carrier 202 can be formed from an elastomeric body material (e.g., an elastomeric body material that is less hard than the material from which the stabilizer 208 was formed). The elastomeric body material of the carrier 202 can be formed around substantially all of the stabilizer 208. This operation 622 can include positioning the stabilizer 208 within a mold used to form the carrier 202, and forming the carrier 202 around at least a portion of the stabilizer 208. Where the stabilizer 208 includes the protrusions 296, the protrusions 296 can facilitate positioning the stabilizer 208 in the mold used to form the carrier 202. The elastomeric body material of the carrier 202 can cover all of the stabilizer 208 except for the areas of the stabilizer 208 having the protrusions 296.

In some examples (e.g., examples in which the stabilizer 208 is formed after forming the carrier 202), operation 620 includes operation 624. Operation 624 includes forming the carrier 202 to have a lumen. The lumen can be sized and shaped to facilitate forming the stabilizer 208 within the carrier 202. The lumen can be formed by forming the carrier 202 around a component having a desired shape for the lumen. Following operation 624, the flow can move to operation 632 of operation 630.

Operation 630 includes disposing the stabilizer 208 in at least a portion of the carrier 202. The stabilizer 208 can be at least partially formed from a second elastomeric body material having a second hardness greater than the first hardness. In some examples, this operation 630 is achieved by encapsulating the stabilizer in the carrier as described in operation 622.

In some examples, operation 630 can include operation 632. Operation 632 includes flowing an elastomeric material into the lumen. Following operation 632, the flow can move to operation 634, which includes curing the elastomeric material.

The process 600 can include further operations to form components having characteristics described elsewhere herein.

As should be appreciated, while particular uses of the technology have been illustrated and discussed above, the disclosed technology can be used with a variety of devices in accordance with many examples of the technology. The above discussion is not meant to suggest that the disclosed technology is only suitable for implementation within systems akin to that illustrated in the figures. For examples, while certain technologies described herein were primarily described in the context of auditory prostheses (e.g., cochlear implants), technologies disclosed herein are applicable to medical devices generally (e.g., medical devices providing pain management functionality or therapeutic electrical stimulation, such as deep brain stimulation). In general, additional configurations can be used to practice the processes and systems herein and/or some aspects described can be excluded without departing from the processes and systems disclosed herein. Further, the techniques described herein can be applicable to determining a recipient's response to other stimuli, such as visual stimuli, tactile stimuli, olfactory stimuli, taste stimuli, or another stimuli. Likewise, the devices used herein need not be limited to auditory prostheses and can be other medical devices configured to support a human sense, such as bionic eyes.

This disclosure described some aspects of the present technology with reference to the accompanying drawings, in which only some of the possible aspects were shown. Other aspects can, however, be embodied in many different forms and should not be construed as limited to the aspects set forth herein. Rather, these aspects were provided so that this disclosure was thorough and complete and fully conveyed the scope of the possible aspects to those skilled in the art.

As should be appreciated, the various aspects (e.g., portions, components, etc.) described with respect to the figures herein are not intended to limit the systems and processes to the particular aspects described. Accordingly, additional configurations can be used to practice the methods and systems herein and/or some aspects described can be excluded without departing from the methods and systems disclosed herein.

Similarly, where steps of a process are disclosed, those steps are described for purposes of illustrating the present methods and systems and are not intended to limit the disclosure to a particular sequence of steps. For example, the steps can be performed in differing order, two or more steps can be performed concurrently, additional steps can be performed, and disclosed steps can be excluded without departing from the present disclosure. Further, the disclosed processes can be repeated.

Although specific aspects were described herein, the scope of the technology is not limited to those specific aspects. One skilled in the art will recognize other aspects or improvements that are within the scope of the present technology. Therefore, the specific structure, acts, or media are disclosed only as illustrative aspects. The scope of the technology is defined by the following claims.

## Claims

1. An apparatus (200) comprising a flexible elongate carrier (202) for introducing a therapeutic element into a recipient, the flexible elongate carrier (202) comprising:
a first elastomeric body material having a first hardness;
a stabilizer (208) extending through at least a portion of the first elastomeric body material,
wherein the stabilizer (208) comprises a second elastomeric body material having a second hardness greater than the first hardness.

2. The apparatus (200) of claim 1,
wherein the first hardness is less than or equal to 60 durometer type A hardness; and
wherein the second hardness is greater than the first hardness and less than or equal to 80 durometer type A hardness.

3. The apparatus (200) of any one of claims 1 or 2, wherein the second elastomeric body material comprises one or more protrusions (296) configured to facilitate centering of the stabilizer (208) within a molding die during manufacturing of the flexible elongate carrier (202).

4. The apparatus (200) of any one of claims 1-3, further comprising:
a plurality of electrodes (212), each respective electrode (212) having a wire (252) extending therefrom for electrically connecting the respective electrode (212) to a device,
wherein, at a point along the flexible elongate carrier (202), the stabilizer (208) has a profile defining a concavity (412); and
wherein at least one of the wires (252) is embedded within the first elastomeric body material and within the concavity (412).

5. The apparatus (200) of any one of claims 1-4, wherein the flexible elongate carrier (202) lacks a metallic stiffener.

6. The apparatus (200) of claim 1,
wherein the stabilizer (208) is permanently embedded within the flexible elongate carrier (202);
wherein the second hardness is 10%, 15%, 20%, 25%, 30%, 35%, 40%, 45%, 50%, 55%, 60%, 65%, 70%, 75%, 80%, 85%, 90%, 95%, or 100% harder than the first hardness;
wherein the stabilizer (208) takes up a percentage of the area of a portion of the carrier (202) in a cross section perpendicular to the long axis of the carrier 202 that is at least x%, where x is at least 10, at least 20, at least 30, at least 40, at least 50, at least 60, at least 70, at least 80, or at least 90;
wherein the flexible elongate carrier (202) comprises a collar (204) configured to resist the introduction of the flexible elongate carrier (202) into the recipient proximally beyond the collar (204); or
wherein a distance (D1) from a beginning of a first electrode (212) of the flexible elongate carrier (202) to an end of a last electrode (212) of the flexible elongate carrier (202) is at least 19 mm.

7. The apparatus (200) of claim 1, wherein:
the flexible elongate carrier (202) has a proximal region (228); and
the stabilizer (208) is permanently embedded in and longitudinally extending through at least the proximal region (228),
wherein the stabilizer (208) is configured to decrease flexibility of the proximal region (228) so as to resist deformation of the proximal region (228) during introduction of the flexible elongate carrier (202) into the recipient.

8. The apparatus of claim 7, wherein the stabilizer (208) is configured to variably decrease flexibility of the proximal region (228).

9. The apparatus of claim 8, wherein the stabilizer (208) has a tapered profile, thereby variably decreasing flexibility of the proximal region (228).

10. The apparatus of any one of claims 7-9, wherein the stabilizer (208) defines one or more flex structures (292) configured to promote bending of the stabilizer (208) in predetermined locations.

11. The apparatus of claim 10, wherein the one or more flex structures (292) include one or more holes or grooves.

12. The apparatus of any one of claims 7-11, wherein the stabilizer (208) defines one or more integration structures (294) configured to provide positive mechanical integration with the flexible elongate carrier (202).

13. The apparatus of any one of claims 7-12, wherein the apparatus further comprises a metallic stiffener disposed within the stabilizer (208).

14. The apparatus of any one of claims 7-13, further comprising:
a handle (206),
wherein the stabilizer (208) extends into the handle (206) and provides stiffness to the handle (206).

15. The apparatus of claim 7,
wherein the apparatus further comprises a collar (204) configured to resist the introduction of the flexible elongate carrier (202) into the recipient proximally beyond the collar (204) and wherein the stabilizer (208) extends proximally beyond the collar;
wherein the apparatus further comprises a metallic stiffener disposed within the stabilizer (208) without extending distally past a collar (204) of the apparatus;
wherein the apparatus further comprises a metallic stiffener that extends from a handle of the apparatus to a marker that indicates a recommended maximum insertion depth;
wherein the apparatus further comprises a metallic stiffener that does not extend to a distal end of the stabilizer;
wherein the therapeutic element comprises at least one electrode (212);
wherein the apparatus is a cochlear implant, a prosthetic hearing implant, neurostimulator cardiac pacemaker, cardiac defibrillator, sleep apnea management stimulator, seizure therapy stimulator, vestibular implant, or bionic eye;
wherein the stabilizer (208) comprises multiple grades of elastomers of increasing durometer from a distal to a proximal end of the stabilizer (208);
wherein the stabilizer (208) lacks insulation;
wherein the stabilizer (208) further comprises a metallic stiffener;
wherein the carrier (202) comprises an elastomeric material that is less hard than the elastomeric material of the stabilizer (208); or
wherein the stabilizer (208) is configured to elastically deform if accidentally bent or buckled during implantation.

16. A method (600) of fabricating an apparatus according to any of the claims 1 to 16, comprising:
forming (620) a carrier (202) at least partially from a first elastomeric body material having a first hardness;
disposing a stabilizer (208) in at least a portion of the carrier (202), wherein the stabilizer (208) is at least partially formed from a second elastomeric body material having a second hardness greater than the first hardness.

17. The method (600) of claim 16, further comprising:
forming (610) the stabilizer (208) prior to forming the carrier (202); and
wherein disposing the stabilizer (208) in at least a portion of the carrier (202) comprises encapsulating (622) the stabilizer (208) in the carrier (202).

18. The method of claim 16,
wherein forming the carrier (202) includes forming the carrier (202) to have a lumen; and
wherein disposing the stabilizer (208) in at least a portion of the carrier (202) includes:
flowing (632) the second elastomeric body material into the lumen; and
curing (634) the second elastomeric body material within the lumen.

19. The method of any one of claims 16-18, further comprising:
at least partially forming the stabilizer (208) from the second elastomeric body material having the second hardness; and
at least partially forming the stabilizer (208) from a plurality of additional elastomeric body materials, each additional elastomeric body material having a different hardness greater than the second hardness.

20. The method of claim 16,
wherein the stabilizer (208) includes one or more protrusions (296) to facilitate centering the stabilizer (208) within a molding die for the carrier (202);
wherein the stabilizer is formed in a single forming process;
wherein the first second elastomeric body material is silicone;
wherein the second elastomeric body material is a medical grade elastomeric material;
wherein the first hardness is less than or equal to 60 durometer type A hardness;
wherein the second hardness is greater than the first hardness and less than or equal to 80 durometer type A hardness;
wherein the elastomeric material has a tensile strength of at least 1030 PSI, an elongation of at least 265%, and a tear resistance of at least 90 PPI; or
wherein the second hardness is 10%, 15%, 20%, 25%, 30%, 35%, 40%, 45%, 50%, 55%, 60%, 65%, 70%, 75%, 80%, 85%, 90%, 95%, or 100% harder than the first hardness.

## Patentansprüche

1. Vorrichtung (200) mit einem flexiblen, länglichen Träger (202) zum Einführen eines therapeutischen Elements in einen Empfänger, wobei der flexible, längliche Träger (202) umfasst:
ein erstes elastomeres Körpermaterial mit einer ersten Härte;
einen Stabilisator (208), der sich durch mindestens einen Teil des ersten elastomeren Körpermaterials erstreckt,
wobei der Stabilisator (208) ein zweites elastomeres Körpermaterial umfasst, das eine zweite Härte aufweist, die größer ist als die erste Härte.

2. Vorrichtung (200) nach Anspruch 1,
wobei die erste Härte kleiner oder gleich 60 Durometer Härte Typ A ist; und
wobei die zweite Härte größer ist als die erste Härte und kleiner oder gleich 80 Durometer Härte Typ A ist.

3. Vorrichtung (200) nach einem der Ansprüche 1 oder 2, wobei das zweite elastomere Körpermaterial einen oder mehrere Vorsprünge (296) aufweist, die so konfiguriert sind, dass sie das Zentrieren des Stabilisators (208) innerhalb einer Gussform während der Herstellung des flexiblen länglichen Trägers (202) erleichtern.

4. Vorrichtung (200) nach einem der Ansprüche 1 bis 3, ferner umfassend:
eine Vielzahl von Elektroden (212), wobei jede jeweilige Elektrode (212) einen Draht (252) aufweist, der sich von ihr aus erstreckt, um die jeweilige Elektrode (212) mit einer Vorrichtung elektrisch zu verbinden,
wobei der Stabilisator (208) an einem Punkt entlang des flexiblen länglichen Trägers (202) ein Profil aufweist, das eine Konkavität (412) definiert; und
wobei mindestens einer der Drähte (252) in das erste elastomere Körpermaterial und in die Konkavität (412) eingebettet ist.

5. Vorrichtung (200) nach einem der Ansprüche 1 bis 4, wobei der flexible, längliche Träger (202) keine metallische Versteifung aufweist.

6. Vorrichtung (200) nach Anspruch 1,
wobei der Stabilisator (208) dauerhaft in den flexiblen, langgestreckten Träger (202) eingebettet ist;
wobei die zweite Härte 10%, 15%, 20%, 25%, 30%, 35%, 40%, 45%, 50%, 55%, 60%, 65%, 70%, 75%, 80%, 85%, 90%, 95% oder 100% härter ist als die erste Härte;
wobei der Stabilisator (208) einen Prozentsatz der Fläche eines Teils des Trägers (202) in einem Querschnitt senkrecht zur Längsachse des Trägers (202) einnimmt, der mindestens x% beträgt, wobei x mindestens 10, mindestens 20, mindestens 30, mindestens 40, mindestens 50, mindestens 60, mindestens 70, mindestens 80 oder mindestens 90 ist;
wobei der flexible längliche Träger (202) einen Kragen (204) umfasst, der so konfiguriert ist, dass er dem Einführen des flexiblen länglichen Trägers (202) in den Empfänger proximal jenseits des Kragens (204) widersteht; oder
wobei ein Abstand (D1) von einem Anfang einer ersten Elektrode (212) des flexiblen langgestreckten Trägers (202) zu einem Ende einer letzten Elektrode (212) des flexiblen langgestreckten Trägers (202) mindestens 19 mm beträgt.

7. Vorrichtung (200) nach Anspruch 1, wobei:
der flexible langgestreckte Träger (202) einen proximalen Bereich (228) aufweist; und
der Stabilisator (208) dauerhaft in mindestens den proximalen Bereich (228) eingebettet ist und sich in Längsrichtung durch diesen erstreckt,
wobei der Stabilisator (208) so konfiguriert ist, dass er die Flexibilität des proximalen Bereichs (228) verringert, um einer Verformung des proximalen Bereichs (228) während der Einführung des flexiblen länglichen Trägers (202) in den Empfänger zu widerstehen.

8. Vorrichtung nach Anspruch 7, wobei der Stabilisator (208) so konfiguriert ist, dass er die Flexibilität des proximalen Bereichs (228) variabel verringert.

9. Vorrichtung nach Anspruch 8, wobei der Stabilisator (208) ein sich verjüngendes Profil aufweist, wodurch die Flexibilität des proximalen Bereichs (228) variabel verringert wird.

10. Vorrichtung nach einem der Ansprüche 7 bis 9, wobei der Stabilisator (208) eine oder mehrere Biegestrukturen (292) definiert, die so konfiguriert sind, dass sie das Biegen des Stabilisators (208) an vorbestimmten Stellen fördern.

11. Vorrichtung nach Anspruch 10, wobei die eine oder mehreren Biegestrukturen (292) ein oder mehrere Löcher oder Nuten aufweisen.

12. Vorrichtung nach einem der Ansprüche 7 bis 11, wobei der Stabilisator (208) eine oder mehrere Integrationsstrukturen (294) definiert, die so konfiguriert sind, dass sie eine positive mechanische Integration mit dem flexiblen, länglichen Träger (202) bereitstellen.

13. Vorrichtung nach einem der Ansprüche 7 bis 12, wobei die Vorrichtung ferner eine metallische Versteifung umfasst, die innerhalb des Stabilisators (208) angeordnet ist.

14. Vorrichtung nach einem der Ansprüche 7 bis 13 umfasst ferner:
einen Griff (206),
wobei der Stabilisator (208) sich in den Griff (206) erstreckt und dem Griff (206) Steifigkeit verleiht.

15. Vorrichtung nach Anspruch 7,
wobei die Vorrichtung ferner einen Kragen (204) umfasst, der so konfiguriert ist, dass er der Einführung des flexiblen länglichen Trägers (202) in den Empfänger proximal über den Kragen (204) hinaus widersteht, und wobei sich der Stabilisator (208) proximal über den Kragen hinaus erstreckt;
wobei die Vorrichtung ferner eine metallische Versteifung umfasst, die innerhalb des Stabilisators (208) angeordnet ist, ohne sich distal über einen Kragen (204) der Vorrichtung hinaus zu erstrecken;
wobei die Vorrichtung ferner eine metallische Versteifung umfasst, die sich von einem Griff der Vorrichtung bis zu einer Markierung erstreckt, die eine empfohlene maximale Einführungstiefe anzeigt;
wobei die Vorrichtung ferner eine metallische Versteifung umfasst, die sich nicht bis zu einem distalen Ende des Stabilisators erstreckt;
wobei das therapeutische Element mindestens eine Elektrode (212) umfasst;
wobei die Vorrichtung ein Cochlea-Implantat, ein prothetisches Hörimplantat, ein Neurostimulator, ein Herzschrittmacher, ein Herzdefibrillator, ein Stimulator zur Behandlung der Schlafapnoe, ein Stimulator zur Anfallstherapie, ein vestibuläres Implantat oder ein bionisches Auge ist;
wobei der Stabilisator (208) mehrere Qualitäten von Elastomeren mit zunehmender Härte von einem distalen zu einem proximalen Ende des Stabilisators (208) umfasst;
wobei der Stabilisator (208) keine Isolierung aufweist;
wobei der Stabilisator (208) ferner eine metallische Versteifung umfasst;
wobei der Träger (202) ein elastomeres Material umfasst, das weniger hart ist als das elastomere Material des Stabilisators (208); oder
wobei der Stabilisator (208) so konfiguriert ist, dass er sich elastisch verformt, wenn er während der Implantation versehentlich gebogen oder geknickt wird.

16. Verfahren (600) zum Herstellen einer Vorrichtung nach einem der Ansprüche 1-15, umfassend:
Formen (620) eines Trägers (202) zumindest teilweise aus einem ersten elastomeren Körpermaterial mit einer ersten Härte;
Anordnen eines Stabilisators (208) in zumindest einem Teil des Trägers (202), wobei der Stabilisator (208) zumindest teilweise aus einem zweiten elastomeren Körpermaterial mit einer zweiten Härte, die größer als die erste Härte ist, gebildet wird.

17. Verfahren (600) nach Anspruch 16, ferner umfassend:
Formen (610) des Stabilisators (208) vor dem Formen des Trägers (202); und
wobei das Anordnen des Stabilisators (208) in mindestens einem Teil des Trägers (202) das Einkapseln (622) des Stabilisators (208) in dem Träger (202) umfasst.

18. Verfahren nach Anspruch 16,
wobei das Ausbilden des Trägers (202) das Ausbilden des Trägers (202) so umfasst, dass er ein Lumen aufweist; und
wobei das Anordnen des Stabilisators (208) in mindestens einem Teil des Trägers (202) umfasst:
Einfließen (632) des zweiten elastomeren Körpermaterials in das Lumen; und
Aushärten (634) des zweiten Elastomerkörpermaterials innerhalb des Lumens.

19. Verfahren nach einem der Ansprüche 16 bis 18, ferner umfassend:
zumindest teilweises Formen des Stabilisators (208) aus dem zweiten elastomeren Körpermaterial mit der zweiten Härte; und
zumindest teilweises Formen des Stabilisators (208) aus einer Vielzahl von zusätzlichen Elastomerkörpermaterialien, wobei jedes zusätzliche Elastomerkörpermaterial eine andere Härte aufweist, die größer ist als die zweite Härte.

20. Verfahren nach Anspruch 16,
wobei der Stabilisator (208) einen oder mehrere Vorsprünge (296) aufweist, um das Zentrieren des Stabilisators (208) innerhalb einer Form für den Träger (202) zu erleichtern;
wobei der Stabilisator in einem einzigen Formgebungsverfahren geformt wird;
wobei das erste elastomere Körpermaterial Silikon ist;
wobei das zweite elastomere Körpermaterial ein medizinisches Elastomermaterial ist;
wobei die erste Härte weniger als oder gleich 60 Durometer Härte Typ A ist;
wobei die zweite Härte größer als die erste Härte und kleiner als oder gleich 80 Durometer Härte Typ A ist;
wobei das elastomere Material eine Zugfestigkeit von mindestens 1030 PSI, eine Dehnung von mindestens 265% und eine Reißfestigkeit von mindestens 90 PPI aufweist; oder
wobei die zweite Härte 10%, 15%, 20%, 25%, 30%, 35%, 40%, 45%, 50%, 55%, 60%, 65%, 70%, 75%, 80%, 85%, 90%, 95% oder 100% härter ist als die erste Härte.

## Revendications

1. Appareil (200) comprenant un support allongé souple (202) pour l'introduction d'un élément thérapeutique chez un receveur, le support allongé souple (202) comprenant :
un premier matériau de corps élastomère ayant une première dureté ;
un stabilisateur (208) s'étendant à travers au moins une portion du premier matériau de corps élastomère,
dans lequel le stabilisateur (208) comprend un second matériau de corps élastomère ayant une seconde dureté supérieure à la première dureté.

2. Appareil (200) selon la revendication 1,
dans lequel la première dureté est inférieure ou égale à une dureté de 60 au duromètre de type A ; et
dans lequel la seconde dureté est supérieure à la première dureté et inférieure ou égale à une dureté de 80 au duromètre de type A.

3. Appareil (200) selon l'une quelconque des revendications 1 ou 2, dans lequel le second matériau de corps élastomère comprend une ou plusieurs protubérances (296) configurées pour faciliter le centrage du stabilisateur (208) à l'intérieur d'une filière de moulage durant la fabrication du support allongé souple (202).

4. Appareil (200) selon l'une quelconque des revendications 1 à 3, comprenant en outre :
une pluralité d'électrodes (212), chaque électrode respective (212) ayant un fil (252) s'étendant depuis celle-ci pour le raccordement électriquement de l'électrode respective (212) à un dispositif,
dans lequel, au niveau d'un point le long du support allongé souple (202), le stabilisateur (208) présente un profil définissant une concavité (412) ; et
dans lequel au moins l'un des fils (252) est enchâssé à l'intérieur du premier matériau de corps élastomère et à l'intérieur de la concavité (412).

5. Appareil (200) selon l'une quelconque des revendications 1 à 4, dans lequel le support allongé souple (202) ne présente pas de raidisseur métallique.

6. Appareil (200) selon la revendication 1,
dans lequel le stabilisateur (208) est enchâssé de manière permanente à l'intérieur du support allongé souple (202) ;
dans lequel la seconde dureté est 10 %, 15 %, 20 %, 25 %, 30 %, 35 %, 40 %, 45 %, 50 %, 55 %, 60 %, 65 %, 70 %, 75 %, 80 %, 85 %, 90 %, 95 %, ou 100 % plus grande que la première dureté ;
dans lequel le stabilisateur (208) prend un pourcentage de la surface d'une portion du support (202) dans une section transversale perpendiculaire à l'axe long du support (202) qui est d'au moins x %, où x est d'au moins 10, d'au moins 20, d'au moins 30, d'au moins 40, d'au moins 50, d'au moins 60, d'au moins 70, d'au moins 80, ou d'au moins 90 ;
dans lequel le support allongé souple (202) comprend un collier (204) configuré pour résister à l'introduction du support allongé souple (202) chez le receveur de manière proximale au-delà du collier (204) ; ou
dans lequel une distance (D1) depuis un début d'une première électrode (212) du support allongé souple (202) jusqu'à une extrémité d'une dernière électrode (212) du support allongé souple (202) est d'au moins 19 mm.

7. Appareil (200) selon la revendication 1, dans lequel :
le support allongé souple (202) présente une région proximale (228) ; et
le stabilisateur (208) est enchâssé de manière permanente à l'intérieur, et s'étend longitudinalement à travers au moins la région proximale (228),
dans lequel le stabilisateur (208) est configuré pour réduire la flexibilité de la région proximale (228) afin de résister à une déformation de la région proximale (228) durant l'introduction du support allongé souple (202) à l'intérieur du receveur.

8. Appareil selon la revendication 7, dans lequel le stabilisateur (208) est configuré pour réduire de manière variable la flexibilité de la région proximale (228).

9. Appareil selon la revendication 8, dans lequel le stabilisateur (208) présente un profil effilé, réduisant ainsi de manière variable la flexibilité de la région proximale (228).

10. Appareil selon l'une quelconque des revendications 7 à 9, dans lequel le stabilisateur (208) définit une ou plusieurs structures souples (292) configurées pour favoriser une flexion du stabilisateur (208) dans des emplacements prédéterminés.

11. Appareil selon la revendication 10, dans lequel la une ou plusieurs structures souples (292) incluent un ou plusieurs trous ou une ou plusieurs rainures.

12. Appareil selon l'une quelconque des revendications 7 à 11, dans lequel le stabilisateur (208) définit une ou plusieurs structures d'intégration (294) configurées pour fournir une intégration mécanique positive avec le support allongé souple (202).

13. Appareil selon l'une quelconque des revendications 7 à 12, dans lequel l'appareil comprend en outre un raidisseur métallique disposé à l'intérieur du stabilisateur (208).

14. Appareil selon l'une quelconque des revendications 7 à 13, comprenant en outre :
une poignée (206),
dans lequel le stabilisateur (208) s'étend dans la poignée (206) et fournit une rigidité à la poignée (206) .

15. Appareil selon la revendication 7,
dans lequel l'appareil comprend en outre un collier (204) configuré pour résister à l'introduction du support allongé souple (202) chez le receveur de manière proximale au-delà du collier (204) et dans lequel le stabilisateur (208) s'étend de manière proximale au-delà du collier ;
dans lequel l'appareil comprend en outre un raidisseur métallique disposé à l'intérieur du stabilisateur (208) sans s'étendre de manière distale au-delà d'un collier (204) de l'appareil ;
dans lequel l'appareil comprend en outre un raidisseur métallique qui s'étend depuis une poignée de l'appareil vers un marqueur qui indique une profondeur d'insertion maximale recommandée ;
dans lequel l'appareil comprend en outre un raidisseur métallique qui ne s'étend pas jusqu'à une extrémité distale du stabilisateur ;
dans lequel l'élément thérapeutique comprend au moins une électrode (212) ;
dans lequel l'appareil est un implant cochléaire, un implant auditif prothétique, un neurostimulateur, un stimulateur cardiaque, un défibrillateur cardiaque, un stimulateur de gestion de l'apnée du sommeil, un stimulateur de thérapie contre les convulsions, un implant vestibulaire, ou un œil bionique ;
dans lequel le stabilisateur (208) comprend des qualités multiples d'élastomères de duromètre croissant depuis une extrémité distale à une extrémité proximale du stabilisateur (208) ;
dans lequel le stabilisateur (208) ne présente pas d'isolation ;
dans lequel le stabilisateur (208) comprend en outre un raidisseur métallique ;
dans lequel le support (202) comprend un matériau élastomère qui est moins dur que le matériau élastomère du stabilisateur (208) ; ou
dans lequel le stabilisateur (208) est configuré pour se déformer élastiquement s'il est accidentellement fléchi ou tordu durant la pose.

16. Procédé (600) de fabrication d'un appareil selon l'une quelconque des revendications 1 à 16, comprenant :
la mise en forme (620) d'un support (202) au moins partiellement depuis un premier matériau de corps élastomère ayant une première dureté ;
la mise en place d'un stabilisateur (208) dans au moins une portion du support (202), dans lequel le stabilisateur (208) est au moins partiellement formé à partir d'un second matériau de corps élastomère ayant une seconde dureté supérieure à la première dureté.

17. Procédé (600) selon la revendication 16, comprenant en outre :
la mise en forme (610) du stabilisateur (208) avant la mise en forme du support (202) ;
et dans lequel la mise en place du stabilisateur (208) dans au moins une portion du support (202) comprend l'encapsulation (622) du stabilisateur (208) dans le support (202).

18. Procédé selon la revendication 16,
dans lequel la mise en forme du support (202) inclut la mise en forme du support (202) pour avoir une lumière ; et
dans lequel la mise en place du stabilisateur (208) dans au moins une portion du support (202) inclut :
l'écoulement (632) du second matériau de corps élastomère dans la lumière ; et
le durcissement (634) du second matériau de corps élastomère à l'intérieur de la lumière.

19. Procédé selon l'une quelconque des revendications 16 à 18, comprenant en outre :
la mise en forme au moins partiellement du stabilisateur (208) à partir du second matériau de corps élastomère ayant la seconde dureté ; et
la mise en forme au moins partiellement du stabilisateur (208) à partir d'une pluralité de matériaux de corps élastomère additionnels, chaque matériau de corps élastomère additionnel ayant une dureté différente supérieure à la seconde dureté.

20. Procédé selon la revendication 16,
dans lequel le stabilisateur (208) inclut une ou plusieurs protubérances (296) pour faciliter le centrage du stabilisateur (208) à l'intérieur d'une filière de moulage pour le support (202) ;
dans lequel le stabilisateur est formé selon un procédé de mise en forme unique ;
dans lequel le premier second matériau de corps élastomère est une silicone ;
dans lequel le second matériau de corps élastomère est un matériau élastomère de qualité médicale ;
dans lequel la première dureté est inférieure ou égale à une dureté de 60 au duromètre de type A ;
dans lequel la seconde dureté est supérieure à la première dureté et inférieure ou égale à une dureté de 80 au duromètre de type A ;
dans lequel le matériau élastomère présente une résistance à la traction d'au moins 1 030 lb/po², un allongement d'au moins 265 %, et une résistance à la déchirure d'au moins 90 PPI ; ou
dans lequel la seconde dureté est 10 %, 15 %, 20 %, 25 %, 30 %, 35 %, 40 %, 45 %, 50 %, 55 %, 60 %, 65 %, 70 %, 75 %, 80 %, 85 %, 90 %, 95 %, ou 100 % plus grande que la première dureté.
